# EUROPEAN PATENT SPECIFICATION

(11) **EP 3 342 457 B1**
(45) Date of publication and mention of the grant of the patent: **04.03.2020**
(21) Application number: 17161222.9
(22) Date of filing: 15.03.2017
(51) Int. Cl.: A61B 18/22, A61B 18/24, A61B 18/26, A61B 1/307, A61B 17/00, A61B 18/00, A61B 1/00

(54) **URETEROSCOPE WITH A LASER AND PROCESSOR-READABLE MEDIUM COMPRISING INSTRUCTIONS FOR DUSTING STONES IN A BODY CAVITY**
URETEROSKOP MIT EINER LASER UND PROZESSOR-LESBARES MEDIUM MIT ANWEISUNGEN ZUM ZERSTÄUBEN VON STEINEN IN EINER KÖRPERHÖHLE
URETÈROSCOPE AVEC UN LASER ET UN SUPPORT LISIBLE PAR PROCESSEUR COMPRENANT DES INSTRUCTIONS POUR POUDRAGE DE CALCULS DANS UNE CAVITÉ CORPORELLE

(30) Priority: 30.12.2016 IN 201641044978
(43) Date of publication of application: 04.07.2018
(73) Proprietor: Wipro Limited, 560 035 Karnataka (IN)
(72) Inventor: MISRA, Pavan, 560094 Bangalore (IN); SELVAM, Gunalan, 560035 Bangalore (IN); BABU, Brijesh, 560016 Bangalore (IN)
(74) Representative: Finnegan Europe LLP

(56) References cited:
- JP-A- 2001 046 395
- US-A1- 2002 161 358
- US-A1- 2014 243 849

## Description

### FIELD OF THE DISCLOSURE

The present subject matter generally relates to the field of ureteroscopes. More particularly, the present disclosure discloses a ureteroscope and a processor-readable medium comprising instructions that, when executed by the processing module of the ureteroscope, cause the processing module to perform a method for dusting stones in a body cavity with a laser fiber.

### BACKGROUND

Ureteroscopy is a method used in treatment of ureteral stones and kidney stones using ureteroscopes. The ureteroscopes have reduced in size and become technologically advanced to facilitate ureteroscopic management of the ureteral stones and the kidney stones.

Currently, a urologist is required to manually feed or retract a laser fiber into a ureteroscope after inserting the ureteroscope into a natural path of a patient to a target stone either in a ureter or a kidney. An assistant is required to hold the ureteroscope during feeding or retracting of the laser fiber by the urologist. In case the assistant moves the ureteroscope, the urologist loses sight of the stone and will again have to pass the laser fiber to somebody else to hold, maneuver the ureteroscope to get a clear vision of the stone and then restart the ureteroscopy using the laser fiber as mentioned above. The urologist further may perform a method of physically twitching and tweaking a laser fiber to break the stone. Such a method is also referred to as dusting. Dusting refers to a process in which a deflecting tip of the ureteroscope having the laser fiber is swayed from one side to another and a low energy high frequency laser pulse is used to break down the stone into a fine dust, without breaking the stone into big fragments. The fine dust is washed away, by saline flowing in through the ureteroscope, out of a urinary system of the patient through the natural path. Such a method of dusting with the laser fiber can be a tedious task and also adds to a load on the urologist and the patient due to the long duration of the dusting process. Such a procedure of dusting also has an increased probability of damaging internal tissues of the urinary system if performed incorrectly or due to poor control of the deflection of the deflecting tip. Moreover, longer procedure time can also affect post-op recovery times and the clinical outcome of the procedure.

With the urologist manually managing the laser fiber by physically twitching and tweaking the laser fiber, there are chances of breakage of the laser fiber which is brittle due to inconsistent force being applied on the laser fiber. If the laser fiber breaks, the urologist has to remove the laser fiber from the ureteroscope and insert another laser fiber, which may happen repeatedly. Such repeated replacement of the laser fiber is costly, as laser fibers are expensive.

JP 2001-046395 A is discloses a laser irradiation device with an elongated main body, a freely bendable curved portion provided at or attached to the distal end of the main body and freely slidable inside the main body, and an optical fiber positioned inside the bendable curved portion, wherein the optical fiber emits a laser beam from the tip sideward or obliquely and wherein bending of the bendable curved portion enables manipulation of the laser beam emitted from the optical fiber.

US 2014/0243849 A1 discloses a robotic manipulator that is capable of controlling in/out movements of laser fiber operably seated inside a working channel of a conventional flexible endoscope and a control console that checks a real-time position of the laser fiber tip and inhibits laser firing if the tip of the laser fiber is close to the distal end of flexible endoscope.

### SUMMARY

According to aspects of the present disclosure, there are provided a ureteroscope for dusting stones in a body cavity with a laser fiber and a processor-readable medium as claimed in claims 1 and 7 respectively. Embodiments of present disclosure disclose a ureteroscope for dusting stones in a body cavity with a laser fiber. The ureteroscope includes a flexible probe and a handle. The handle extends proximally from the flexible probe. The flexible probe includes a proximal end and a distal end. The flexible probe includes a working channel that is configured to accommodate a laser fiber in the distal end of the flexible probe at a working position. The laser fiber is deflected against a target stone for dusting, the working position being a predefined distance of the laser fiber from the distal end of the flexible probe and defined by laser energy setting and composition of the target stone. The handle includes a plurality of control switches and a laser fiber feeder and retraction module. The plurality of control switches is configured to provide digital control signals. The digital control signals are configured to control a motor assisted movement of the laser fiber in the working channel by a user. The motor assisted movement of the laser fiber includes one of a feeding movement and a retracting movement. The laser fiber feeder and retraction module is configured to control one of the feeding movement and the retracting movement of the laser fiber in the working channel. The feeding movement of the laser fiber includes a forward movement of the laser fiber in a first rotating direction and the retracting movement of the laser fiber includes a backward movement of the laser fiber in a second rotating direction.

Disclosed herein is an exemplary method of dusting stones in a body cavity with a laser fiber in a ureteroscope. The method includes receiving, by a processing module of a laser fiber feeder and retraction module of the ureteroscope, a digital light dependent resistor signal from a light dependent resistor (LDR) and digital control signals from a plurality of control switches. The digital LDR signal is generated in response to sensing presence of a laser fiber in a working channel of a flexible probe of the ureteroscope. The laser fiber is manually introduced into the working channel and positioned at an initial position and configured to be advanced to a working position. The digital control signals are configured to control a motor assisted movement of the laser fiber in the working channel by a user. The motor assisted movement of the laser fiber includes one of a feeding movement and a retracting movement. The method also includes processing, by the processing module, the digital LDR signal and the digital control signals to generate an analog motor signal. Further, the method includes initiating, by the processing module, activation of a motor in the laser fiber feeding and retraction module in response to the analog motor signal. The motor controls one of the feeding movement and the retracting movement of the laser fiber in the working channel for dusting of a target stone. The feeding movement of the laser fiber includes a forward movement of the laser fiber in a first rotating direction, and the retracting movement of the laser fiber includes a backward movement of the laser fiber in a second rotating direction. Further disclosed herein is a processor-readable medium comprising instructions that, when executed by the processing module of the ureteroscope, cause the processing module to perform the exemplary method.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

The accompanying drawings, which are incorporated in and constitute a part of this disclosure, illustrate exemplary embodiments and, together with the description, serve to explain the disclosed principles. In the figures, the left-most digit(s) of a reference number identifies the figure in which the reference number first appears. The same numbers are used throughout the figures to reference like features and components. Some embodiments of system and exemplary methods are now described, by way of example only, and with reference to the accompanying figures, in which:
FIG. 1 illustrates a side view of a ureteroscope for dusting stones in a body cavity with a laser fiber, in accordance with some embodiments of the present disclosure;
FIG. 2 illustrates a cross-sectional view of a deflecting tip of a ureteroscope, in accordance with some embodiments of the present disclosure;
FIG. 3 illustrates a side view of a ureteroscope for dusting stones in a body cavity with a laser fiber, in accordance with other embodiments of the present disclosure;
FIG. 4 illustrates a block diagram of a processing module of a laser fiber feeder and retraction module in a ureteroscope, in accordance with some embodiments of the present disclosure; and
FIG. 5 is a flow diagram illustrating an exemplary method of dusting stones in a body cavity with a laser fiber in a ureteroscope, in accordance with some examples of the present disclosure.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

### DETAILED DESCRIPTION

In the present document, the word "exemplary" is used herein to mean "serving as an example, instance, or illustration." Any embodiment or implementation of the present subject matter described herein as "exemplary" is not necessarily to be construed as preferred or advantageous over other embodiments.

While the disclosure is susceptible to various modifications and alternative forms, specific embodiment thereof has been shown by way of example in the drawings and will be described in detail below. It should be understood, however that it is not intended to limit the disclosure to the particular forms disclosed, but on the contrary, the disclosure is to cover all modifications, equivalents, and alternative falling within the scope of the disclosure, the scope of the invention being defined by the appended claims.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises... a" does not, without more constraints, preclude the existence of other elements or additional elements in the system or apparatus.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure, the scope of the invention being limited only by the appended claims. The following description is, therefore, not to be taken in a limiting sense.

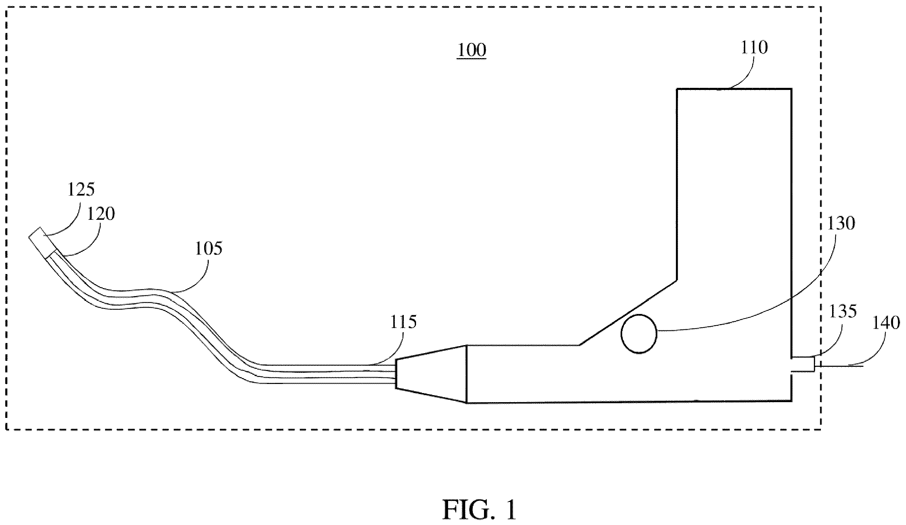

FIG. 1 illustrates a side view of a ureteroscope 100 for dusting stones in a body cavity with a laser fiber, in accordance with some embodiments of the present disclosure.

The ureteroscope 100 includes a flexible probe 105 and a handle 110. The handle 110 extends proximally from the flexible probe 105. The flexible probe 105 includes a proximal end 115 and a distal end 120. The proximal end 115 of the flexible probe 105 is coupled to the handle 110. The flexible probe 105 includes at least one working channel and a deflecting tip 125. The deflecting tip 125 of the flexible probe 105 is coupled to the distal end 120. The flexible probe 105 further includes an image viewing system (not shown in FIG. 1) and a light source (not shown in FIG. 1). The handle 110 includes a plurality of control switches, for example a control switch 130, on an exterior portion of the handle 110. The handle 110 also includes a working channel 135, on an exterior portion of the handle 110, to receive a laser fiber 140. The working channel 135 continues into the flexible probe 105. The handle 110 further includes a laser fiber feeder and retraction module (not shown in FIG. 1) in an interior portion of the handle 110.

The ureteroscope 100 can be used in a stone removal operation from a kidney of a patient. A user, for example a doctor or other medical personnel, inserts the flexible probe 105 of the ureteroscope 100 into a urethra of the patient. The user advances the flexible probe 105 such that the deflecting tip 125 passes into and through a urinary bladder, into and through a ureter, and into a kidney of the patient.

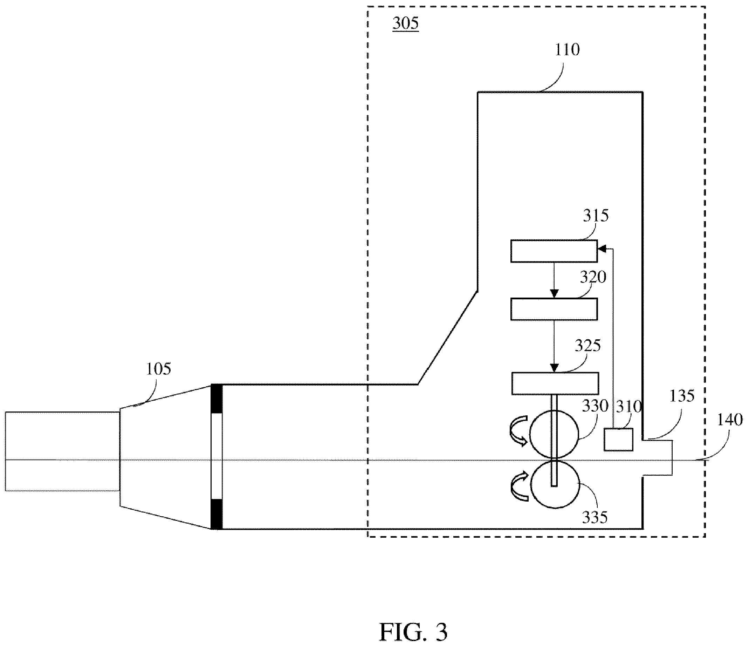

The user positions the deflecting tip 125 of the ureteroscope 100 within the kidney at a minimum distance from a target stone. Once the deflecting tip 125 of the ureteroscope 100 is positioned in the kidney, the laser fiber 140 is received into the working channel 135 of the flexible probe 105 at an initial position and advanced into the working channel 135. Herein, the 'initial position' is referred to as a predefined distance of the laser fiber 140 in the working channel 135 prior to a feeding movement of the laser fiber 140 by the laser fiber feeder and retraction module. The laser fiber 140 is accommodated in the distal end 120 of the flexible probe 105 at a working position. In an example the laser fiber 140 may include a diameter of 200 micrometer, 272 micrometer, 365 micrometer, 550 micrometer, or 1000 micrometer. Herein, the 'working position' is referred to as a predefined distance of the laser fiber 140 from the distal end 120 of the flexible probe 105 defined by laser energy settings and stone composition. The user operates the control switch 130 to control a motor assisted movement of the laser fiber 140 in the working channel 135 by the user. The motor assisted movement of the laser fiber 140 includes one of the feeding movement and a retracting movement of the laser fiber 140 in the working channel 135. The feeding movement and the retracting movement of the laser fiber 140 is described in detail with reference to FIG. 3.

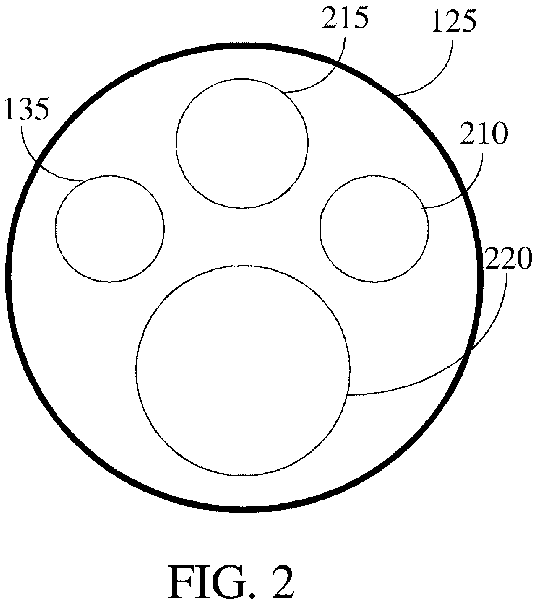

In some examples, the ureteroscope 100 may also be positioned in a ureter of the patient for dusting the target stone in the ureter with the laser fiber 140. The deflecting tip 125 including the working channel 135 is explained in detail with reference to FIG. 2.

Referring now to FIG. 2, a cross sectional view of the deflecting tip 125 including the working channel 135 is illustrated, in accordance with some examples of the present disclosure. In some embodiments, the deflecting tip 125 may be composed of a plurality of segmented portions held together with a plurality of control wires such that the deflecting tip 125 is flexible. As illustrated in FIG. 2, the deflecting tip 125 includes the working channel 135 and a working channel 210. The working channel 135 is configured to accommodate the laser fiber 140 for dusting the target stone. In some examples, the working channel 135 may accommodate a 3 french gauge (Fr) size working device. In other examples, the working channel 135 may be used to accommodate baskets, grasping forceps, and the like. The working channel 210 is configured to be used for irrigation, for example with saline, to wash away dust after the dusting of the target stone with the laser fiber 140.

The deflecting tip 125 further includes an image viewing system 215 and a light source 220. In one example, the image viewing system 215 is a complementary metal oxide semiconductor (CMOS) based image viewing system. The image viewing system 215 is configured to view the laser fiber 140 extending from the deflecting tip 125 and also the target stone. The light source 220 is configured to provide light to assist viewing of the target stone and the laser fiber 140 through the image viewing system 215.

The feeding movement and the retraction movement of the laser fiber 140 in the ureteroscope 100 and subsequent dusting using the laser fiber 140 is explained in detail with reference to FIG. 3.

FIG. 3 illustrates a side view of the ureteroscope 100 for dusting stones in the body cavity of the patient with the laser fiber 140, in accordance with other embodiments of the present disclosure. In the illustrated FIG. 3, a laser fiber feeder and retraction module 305 of the handle 110 and the dusting of the target stone with the laser fiber 140 are described herein in detail. The laser fiber feeder and retraction module 305 is configured to control one of the feeding movement and the retracting movement of the laser fiber 140 in the working channel 135. The feeding movement of the laser fiber 140 includes a forward movement of the laser fiber 140 in a first rotating direction. The retracting movement of the laser fiber 140 includes a backward movement of the laser fiber 140 in a second rotating direction.

The handle 110 includes the laser fiber feeder and retraction module 305 (an electromechanical module) in the interior portion of the handle 110. The laser fiber feeder and retraction module 305 includes a light dependent resistor (LDR) 310, a processing module 315, a driver 320, a motor 325, and a plurality of pulleys, for example a first pulley 330 and a second pulley 335.

The flexible probe 105 of the ureteroscope 100 is inserted into the body cavity of the patient and the defecting tip 125 is deflected or maneuvered to reach a position of the target stone in the kidney or the ureter of the patient. The laser fiber 140 is then inserted into the working channel 135. The feeding movement and the retracting movement of the laser fiber 140 using the laser fiber feeder and retraction module 305 is explained below.

In the feeding movement, the laser fiber 140 is manually introduced into the working channel 135 and positioned at the initial position. The LDR 310 senses presence of the laser fiber 140 in the working channel 135. The LDR 310 then generates a digital LDR signal in response to sensing presence of the laser fiber 140 in the working channel 135. The LDR 310 triggers the processing module 315 with the digital LDR signal to activate the motor 325. The motor 325 rotates a small step in order to grip the laser fiber 140 at the initial position.

In order to feed the laser fiber 140 further into the working channel 135, the user can press the control switch 130 to generate the digital control signals. The processing module 315 coupled to the LDR 310 generates a digital motor signal in response to the digital LDR signal and the digital control signals. The driver 320 is coupled to the processing module 315 and generates an analog motor signal in response to the digital motor signal. The motor 325, for example a stepper motor, is coupled to the driver 320. The motor 325 is configured to turn a number of steps based on the analog motor signal at a preset speed. In an example, the motor 325 may be associated with a step angle of 1.8 degrees, a holding torque of 14 Newton-centimeters, and a rotor inertia of 0.012 kilogramme square centimeter. The plurality of pulleys, for example the first pulley 330 and the second pulley 335, is coupled to the motor 325. In an example, the plurality of pulleys include biocompatible elastic polyurethane over-mold on biocompatible plastic pulleys for gripping and feeding laser fibers of various diameters without slipping. In an example, the plurality of pulleys is over-molded with compressible rubber grippers to grip the laser fiber during the feeding and retracting of the laser fiber in presence of saline flowing through the working channel 210 during the dusting. The compressible rubber grippers permit the user to select between different sizes of laser fibers available for the dusting as determined by energy levels required based on kidney stone composition and sizes.

The plurality of pulleys is configured to control the motor assisted movement of the laser fiber 140 in the working channel. The plurality of pulleys push the laser fiber 140 into the working channel 135 in the forward movement of the laser fiber 140 in the first rotating direction, for example a clockwise direction. The laser fiber 140 is pushed until the working position is reached or a predefined position from the deflecting tip 125 is reached. The dusting of the target stone is further performed using the laser fiber 140 at the working position.

In some examples, the plurality of pulleys is included in a feeder housing that is a biocompatible plastic enclosure for mounting the plurality of pulleys.

In some examples, another control switch may be pressed by the user in order to achieve finer steps or position the laser fiber 140 in an appropriate position during the feeding movement. The processing module 315 instructs the motor 325 accordingly.

Subsequent to the feeding movement of the laser fiber 140 and positioning of the laser fiber 140 at the working position, the laser fiber 140 is energized to a power level and a frequency level to enable the dusting of the target stone. The laser fiber 140 can then be retracted in the retracting movement explained below.

In the retracting movement, the laser fiber 140 is already present in the working channel 135 and is positioned at the working position or the predefined position from the deflecting tip 125.

In order to retract the laser fiber 140 from the working channel 135, the user can press another control switch to trigger processing module 315 to activate the motor 325. The motor 325 rotates a small step in a reverse direction. The plurality of pulleys hence retract the laser fiber 140 from the working channel 135 in the backward movement of the laser fiber 140 in the second rotating direction, for example an anti-clockwise direction. The laser fiber 140 is retracted until the initial position is reached. The laser fiber 140 may further be manually pulled out by the user.

In some examples, another control switch may be pressed by the user in order to achieve finer steps or position the laser fiber 140 in an appropriate position during the retracting movement. The processing module 315 instructs the motor 325 accordingly.

In some examples, a laser trigger is used to energize the laser fiber and is a pedal activated module which is located away from the ureteroscope 100. In other embodiments, the laser trigger may be included within the ureteroscope 100.

In some embodiments, the image viewing system 215 and the light source 220 provides aid to the user to position the laser fiber 140 near the target stone and to perform the dusting of the target stone.

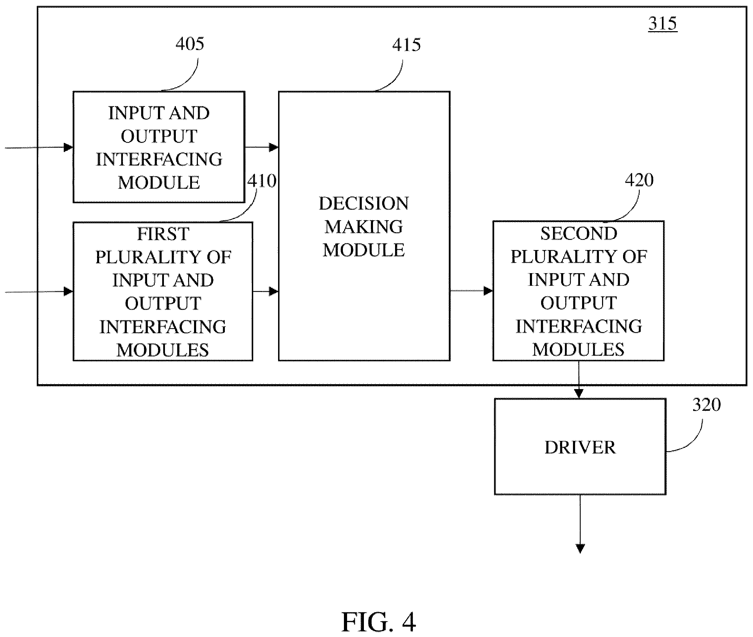

The functions of the processing module 315 during the feeding movement and the retracting movement are explained in detail with reference to FIG. 4.

Referring now to FIG. 4, the processing module 315 is illustrated, in accordance with some embodiments of the present disclosure. The processing module 315 includes an input and output interfacing module 405 and a first plurality of input and output interfacing modules 410. The processing module 315 further includes a decision making module 415. In addition, the processing module 315 includes a second plurality of input and output interfacing modules 420.

The input and output interfacing module 405 and the first plurality of input and output interfacing modules 410 are coupled to the decision making module 415. The decision making module 415 is further coupled to the second plurality of input and output interfacing modules 420. The driver 320 is coupled to the second plurality of input and output interfacing modules 420. An output of the driver 320 is further coupled to the motor 325.

Once the laser fiber 140 is fed into the working channel 135, the LDR 310 senses the presence of the laser fiber 140 and triggers the processing module 315. The input and output interfacing module 405 receives the digital LDR signal from the LDR 310. The digital LDR signal is transmitted to the decision making module 415.

The first plurality of input and output interfacing modules 410 receive the digital control signals from the plurality of control switches, for example the control switch 130. The digital control signals are received when the user selects the control switch 130 or another control switch to control the feeding movement and the retracting movement of the laser fiber 140. The digital control signals are transmitted to the decision making module 415.

The decision making module 415 is configured to determine the presence of the laser fiber 140 in the working channel 135 based on the digital LDR signal, and to determine a type of the feeding movement and the retracting movement of the laser fiber 140 based on the digital control signals. The type of the feeding movement of the laser fiber 140 includes one of a fine feed and a coarse feed of the laser fiber 140. The type of the retracting movement of the laser fiber 140 includes one of a fine retract and a coarse retract of the laser fiber 140.

The second plurality of input and output interfacing modules 420 is configured to receive an output data signal from the decision making module 415. Based on the output data signal received from the decision making module 415, the second plurality of input and output interfacing modules 420 generates the digital motor signal.

The driver 320 receives the digital motor signal from the second plurality of input and output interfacing modules 420 and in turn generates the analog motor signal. The motor 325, coupled to the driver 320, hence turns the number of steps in accordance with the analog motor signal. The motor assisted movement of the laser fiber 140 in the working channel 135 is hence controlled by controlling the plurality of pulleys coupled to the motor 325. The laser fiber 140 is energized after the feeding movement and the dusting of the target stone is performed. The retracting movement of the laser fiber 140 from the working channel 135 is subsequently performed.

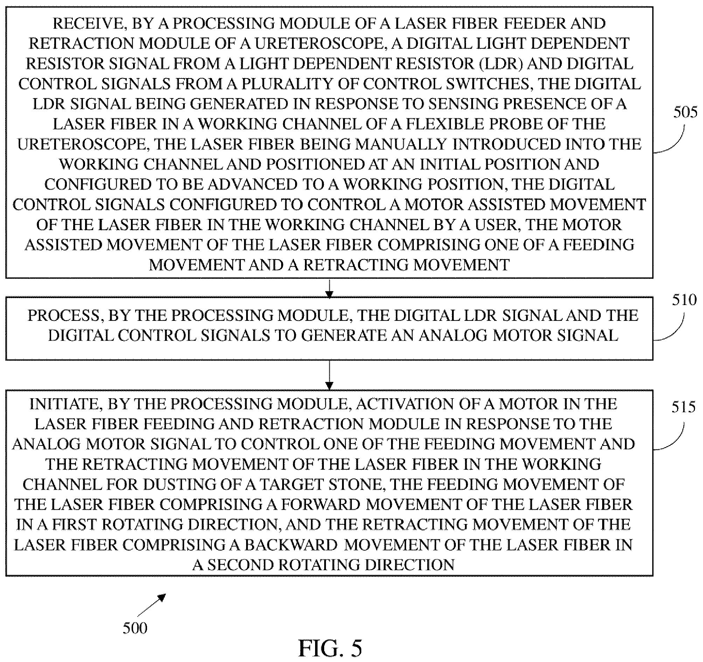

FIG. 5 is a flow diagram illustrating an exemplary method 500 of dusting stones in a body cavity with a laser fiber, for example the laser fiber 140 of FIG. 1, in a ureteroscope, for example the ureteroscope 100 of FIG. 1. The term 'dusting' refers to a technique for disintegrating stones in a kidney or a ureter of a patient into fine fragments with a laser fiber by moving a deflecting tip of the ureteroscope, for example the deflecting tip 125 of the ureteroscope 100 of FIG. 1.

The order in which the method is described is not intended to be construed as a limitation, and any number of the described method blocks can be combined in any order to implement the method. Additionally, individual blocks may be deleted from the method.

At step 505, the method 500 includes receiving a digital light dependent resistor signal from a light dependent resistor (LDR), for example the LDR 310 of FIG. 3, and digital control signals from a plurality of control switches, for example the control switch 130 of FIG. 1. The digital LDR signal and the digital control signals are received by a processing module of a laser fiber feeder and retraction module, for example the processing module 315 of the laser fiber feeder and retraction module 305, of the ureteroscope, The digital LDR signal is generated in response to sensing presence of a laser fiber in a working channel of a flexible probe of the ureteroscope, for example the laser fiber 140 in the working channel 135 of a flexible probe 105. The laser fiber is manually introduced into the working channel and positioned at an initial position and configured to be advanced to a working position.

The initial position is defined as a predefined distance of the laser fiber in the working channel prior to the feeding movement of the laser fiber by the laser fiber feeder and retraction module. The working position is defined as a predefined distance of the laser fiber from a distal end of the flexible probe and is defined by laser energy settings and composition of the target stone.

The digital control signals control a motor assisted movement of the laser fiber in the working channel by a user. The motor assisted movement of the laser fiber includes one of a feeding movement and a retracting movement of the laser fiber. At the working position, the laser fiber extends from a deflecting tip, for example the deflecting tip 125, of the flexible probe. The laser fiber is viewed by an image viewing system, for example the image viewing system 215 of FIG. 2, in the flexible probe with assistance of light provided by a light source, for example the light source 220 of FIG. 2, in the flexible probe.

At step 510, the method 500 includes processing, by the processing module, the digital LDR signal and the digital control signals to generate an analog motor signal. The analog motor signal is generated by the processing module of the laser fiber feed and retraction module. The analog motor signal is further provided to a driver of a motor, for example the driver 320 of the motor 325 of FIG. 3, in the ureteroscope. The method of generating the analog motor signal is explained with reference to FIG. 4 is not explained herein for sake of brevity.

At step 515, the method 500 includes initiating, by the processing module, activation of the motor in the laser fiber feeding and retraction module in response to the analog motor signal. The analog motor signal controls one of the feeding movement and the retracting movement of the laser fiber in the working channel for dusting of a target stone. The feeding movement of the laser fiber includes a forward movement of the laser fiber in a first rotating direction. The retracting movement of the laser fiber includes a backward movement of the laser fiber in a second rotating direction. In an example, the motor used is a stepper motor. The driving of the motor pushes forward or backward the laser fiber using a plurality of pulleys, for example the first pulley 330 and the second pulley 335 of FIG. 3. In an example, the plurality of pulleys are over-molded with compressible rubber grippers to grip the laser fiber during the feeding and retracting of the laser fiber. The method of the feeding movement and the retracting movement of the laser fiber is explained with reference to FIG. 3 is not explained herein for sake of brevity.

The feeding movement and the retracting movement of the laser fiber is controlled by one or more method steps. The method includes generating, by the processing module, an output data signal corresponding to the digital LDR signal and the digital control signals. The output data signal defines the presence of the laser fiber in the working channel based on the digital LDR signal and type of the feeding movement and the retracting movement of the laser fiber.

The method also includes processing, by the processing module, the output data signal to generate the analog motor signal.

The method further includes initiating, by the processing module, driving of the motor to turn a number of steps based on the analog motor signal to move the laser fiber in one of the feeding movement and the retracting movement of the laser fiber using a plurality of pulleys.

The laser fiber extending from the deflecting tip is deflected subsequent to the feeding movement to enable the dusting of the target stone. The laser fiber is energized to a power level and a frequency level to enable the dusting of the target stone. Subsequently, the retracting movement of the laser fiber is performed.

In some examples, the laser fiber is energized using a laser trigger that is a pedal activated module and which is located away from the ureteroscope. In other embodiments, the laser fiber is energized to a power level and a frequency level using the laser trigger included within the ureteroscope.

In some embodiments, the type of the feeding movement and the retracting movement of the laser fiber is determined by the processing module based on the digital control signals. The type of the feeding movement of the laser fiber includes one of a fine feed and a coarse feed of the laser fiber. The type of the retracting movement of the laser fiber includes one of a fine retract and a coarse retract of the laser fiber.

In some examples, after the dusting of the target stone with the laser fiber, dust of the target stone is flushed outside the body using the ureteroscope without affecting adjacent body tissues.

Embodiments of the present disclosure provide a ureteroscope and a processor-readable medium comprising instructions that, when executed by the processing module of the ureteroscope, cause the processing module to perform a method for dusting stones in a body cavity with a laser fiber. The present disclosure provides a controlled and motorized constant rate of feeding or retracting of a laser fiber. Such an exemplary method leads to a reduced risk of laser fiber buckling or kinking that usually result in damages during process of feeding or retraction of the laser fiber. The present disclosure also allows gripping of the laser fiber during feeding and retraction which prevents the laser fiber from, thereby preventing any hygiene issues that might arise through contamination. The present disclosure allows a constant amount of pressure to be applied on the laser fiber resulting in reduced risks of laser fiber breakages during the process of feeding and retracting of the laser fiber. The present disclosure further reduces number of assisting personnel during the dusting and has a systematic and hassle free process of handling the laser fiber during the dusting.

The ureteroscope in the present disclosure is a generic device that may be applied in different flexible ureteroscopes which are used in places where flexible ureteroscopy is performed. The present disclosure of providing an automated motor assisted movement of the laser fiber during feeding and retraction may also be applied to various flexible scopes with a working channel within which a laser fiber or other devices of similar dimensions are required to be fed or retracted. Such flexible scopes are used for different minimally invasive surgeries, for example a laparoscopic procedure.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

The illustrated operations of FIG. 5 show certain events occurring in a certain order. In alternative examples, certain operations may be performed in a different order, modified or removed. Moreover, steps may be added to the above described logic and still conform to the described embodiments. Further, operations described herein may occur sequentially or certain operations may be processed in parallel. Yet further, operations may be performed by a single processing unit or by distributed processing units.

Finally, the language used in the specification has been principally selected for readability and instructional purposes, and it may not have been selected to delineate or circumscribe the subject matter. It is therefore intended that the scope of the invention be limited only by the appended claims. Accordingly, the disclosure of the embodiments of the invention is intended to be illustrative, but not limiting, of the scope of the invention, which is set forth in the following claims.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A ureteroscope (100) for dusting stones in a body cavity with a laser fiber (140), the ureteroscope (100) comprising:
a flexible probe (105) comprising a proximal end (115) and a distal end (120), the flexible probe (105) comprising:
a working channel (135) configured to accommodate the laser fiber (140) in the distal end (120) of the flexible probe (105) at a working position, the laser fiber (140) to be deflected against a target stone for dusting, the working position being a predefined distance of the laser fiber (140) from the distal end (120) of the flexible probe (105) and defined by laser energy settings and composition of the target stone; and
a handle (110) extending proximally from the flexible probe (105), the handle (110) comprising:
a plurality of control switches (130) configured to provide digital control signals, the digital control signals configured to control a motor assisted movement of the laser fiber (140) in the working channel (135) by a user, the motor assisted movement of the laser fiber (140) comprising one of a feeding movement and a retracting movement; and
a laser fiber feeder and retraction module (305) configured to control one of the feeding movement and the retracting movement of the laser fiber (140) in the working channel (135), the feeding movement of the laser fiber (140) comprising a forward movement of the laser fiber (140) in a first rotating direction, and the retracting movement of the laser fiber (140) comprising a backward movement of the laser fiber (140) in a second rotating direction.

2. The ureteroscope (100) as claimed in claim 1, wherein the flexible probe (105) further comprises:
a deflecting tip (125) at the distal end (120) of the flexible probe (105) and configured to be deflected and in turn deflect the laser fiber (140), the deflecting tip (125) being positioned at a minimum distance from the target stone;
an image viewing system (215) configured to view the laser fiber (140) extending from the deflecting tip (125) and the target stone; and
a light source (220) configured to provide light to assist viewing of the laser fiber (140) and the target stone through the image viewing system (215).

3. The ureteroscope (100) as claimed in claim 1 or claim 2, wherein the laser fiber feeder and retraction module (305) comprises:
a light dependent resistor, LDR, (310) configured to generate a digital LDR signal in response to sensing the presence of the laser fiber (140) in the working channel (135), the laser fiber (140) being configured to be manually introduced into the working channel (135) and positioned at an initial position;
a processing module (315) coupled to the LDR (310) and configured to generate a digital motor signal in response to the digital LDR signal and the digital control signals;
a driver (320) coupled to the processing module (315) and configured to generate an analog motor signal in response to the digital motor signal;
a motor (325) coupled to the driver (320) and configured to turn a number of steps based on the analog motor signal; and
a plurality of pulleys (330, 335) coupled to the motor (320) and configured to control the motor assisted movement of the laser fiber (140) in the working channel (135).

4. The ureteroscope (100) as claimed in claim 3, wherein the processing module (315) further comprises:
a controller comprising:
an input and output interfacing module (405) configured to receive the digital LDR signal from the LDR (310);
a first plurality of input and output interfacing modules (410) configured to receive the digital control signals from the plurality of control switches (130);
a decision making module (415) configured to determine the presence of the laser fiber (140) in the working channel (135) based on the digital LDR signal, and to determine a type of the feeding movement and the retracting movement of the laser fiber (140) based on the digital control signals; and
a second plurality of input and output interfacing modules (420) coupled to the decision making module (415) and configured to receive an output data signal from the decision making module (415) and to generate the digital motor signal.

5. The ureteroscope (100) as claimed in claim 3 or claim 4, wherein the initial position is a predefined distance of the laser fiber (140) in the working channel (135) prior to the feeding movement of the laser fiber (140) by the laser fiber feeder and retraction module (305).

6. The ureteroscope (100) as claimed in claim 1, wherein the laser fiber (140) is configured to be energized to a power level and a frequency level to enable the dusting of the target stone.

7. A processor-readable medium comprising instructions that, when executed by the processing module (315) of the ureteroscope (100) as claimed in any of claims 3 to 5, cause the processing module (315) to perform a method for controlling the laser fiber in the ureteroscope (100), the method comprising:
receiving a digital light dependent resistor signal from the LDR (310) and digital control signals from the plurality of control switches (130), the digital LDR signal being generated in response to sensing the presence of the laser fiber (140) in the working channel (135) of the flexible probe (105) of the ureteroscope (100);
processing the digital LDR signal and the digital control signals to generate an analog motor signal; and
initiating activation of the motor (325) in the laser fiber feeding and retraction module (305) in response to the analog motor signal to control one of the feeding movement and the retracting movement of the laser fiber (140) in the working channel (135) for dusting of a target stone.

8. The processor-readable medium as claimed in claim 7, wherein the feeding movement and the retracting movement of the laser fiber (140) is controlled by:
generating an output data signal corresponding to the digital LDR signal and the digital control signals, the output data signal defining the presence of the laser fiber (140) in the working channel (135) based on the digital LDR signal and type of the feeding movement and the retracting movement of the laser fiber (140);
processing the output data signal to generate the analog motor signal; and
initiating driving of the motor (325) to turn a number of steps based on the analog motor signal to move the laser fiber (140) in one of the feeding movement and the retracting movement of the laser fiber (140) using the plurality of pulleys (330, 335).

9. The processor-readable medium as claimed in claim 7 or claim 8, wherein the method further comprises:
determining the type of the feeding movement and the retracting movement of the laser fiber (140) based on the digital control signals, the type of feeding movement of the laser fiber (140) comprising one of a fine feed and a coarse feed of the laser fiber (140), the type of retracting movement of the laser fiber (140) comprising one of a fine retract and a coarse retract of the laser fiber (140).

## Patentansprüche

1. Urethroskop (100) zum Stäuben von Steinen in einer Körperhöhle mit einer Laserfaser (140), wobei das Urethroskop (100) umfasst:
eine flexible Sonde (105), die ein proximales Ende (115) und ein distales Ende (120) umfasst, wobei die flexible Sonde (105) umfasst:
einen Arbeitskanal (135), der konfiguriert ist, um die Laserfaser (140) in dem distalen Ende (120) der flexiblen Sonde (105) an einer Arbeitsposition aufzunehmen, wobei die Laserfaser (140) gegen einen Zielstein zum Stäuben abgelenkt werden soll, wobei die Arbeitsposition ein vordefinierter Abstand der Laserfaser (140) von dem distalen Ende (120) der flexiblen Sonde (105) ist und durch Laserenergieeinstellungen und Zusammensetzung des Zielsteins definiert ist; und
einen Griff (110), der sich proximal von der flexiblen Sonde (105) erstreckt, wobei der Griff (110) umfasst:
eine Vielzahl von Steuerschaltern (130), die konfiguriert ist, um digitale Steuersignale bereitzustellen, wobei die digitalen Steuersignale konfiguriert sind, um eine motorunterstützte Bewegung der Laserfaser (140) in dem Arbeitskanal (135) durch einen Benutzer zu steuern, wobei die motorunterstützte Bewegung der Laserfaser (140) eine von einer Zuführbewegung und einer Rückzugsbewegung umfasst; und
ein Laserfaserzufuhrvorrichtungs- und Rückzugsmodul (305), das konfiguriert ist, um eine von der Zuführbewegung und der Rückzugsbewegung der Laserfaser (140) in dem Arbeitskanal (135) zu steuern, wobei die Zuführbewegung der Laserfaser (140) eine Vorwärtsbewegung der Laserfaser (140) in einer ersten Drehrichtung umfasst und die Rückzugsbewegung der Laserfaser (140) eine Rückwärtsbewegung der Laserfaser (140) in einer zweiten Drehrichtung umfasst.

2. Urethroskop (100) nach Anspruch 1, wobei die flexible Sonde (105) ferner umfasst:
eine Ablenkspitze (125) an dem distalen Ende (120) der flexiblen Sonde (105) und die konfiguriert ist, um abgelenkt zu werden und wiederum die Laserfaser (140) abzulenken, wobei die Ablenkspitze (125) in einem minimalen Abstand von dem Zielstein positioniert ist;
ein Bildbetrachtungssystem (215), das konfiguriert ist, um die Laserfaser (140) zu betrachten, die sich von der Ablenkspitze (125) und dem Zielstein erstreckt; und
eine Lichtquelle (220), die konfiguriert ist, um Licht bereitzustellen, um das Betrachten der Laserfaser (140) und des Zielsteins durch das Bildbetrachtungssystem (215) zu unterstützen.

3. Urethroskop (100) nach Anspruch 1 oder Anspruch 2, wobei das Laserfaserzufuhrvorrichtungs- und Rückzugsmodul (305) umfasst:
einen lichtabhängigen Widerstand (light dependent resisitor - LDR) (310), der konfiguriert ist, um ein digitales LDR-Signal als Reaktion auf das Erfassen des Vorhandenseins der Laserfaser (140) in dem Arbeitskanal (135) zu erzeugen, wobei die Laserfaser (140) konfiguriert ist, um manuell in den Arbeitskanal (135) eingeführt zu werden und in einer Anfangsposition positioniert zu werden;
ein Verarbeitungsmodul (315), das mit dem LDR (310) verbunden ist und konfiguriert ist, um ein digitales Motorsignal als Reaktion auf das digitale LDR-Signal und die digitalen Steuersignale zu erzeugen;
einen Treiber (320), der mit dem Verarbeitungsmodul (315) verbunden ist und konfiguriert ist, um ein analoges Motorsignal als Reaktion auf das digitale Motorsignal zu erzeugen;
einen Motor (325), der mit dem Treiber (320) verbunden ist und konfiguriert ist, um eine Anzahl von Schritten auf Grundlage des analogen Motorsignals zu drehen; und
eine Vielzahl von Scheiben (330, 335), die mit dem Motor (320) verbunden ist und konfiguriert ist, um die motorunterstützte Bewegung der Laserfaser (140) in dem Arbeitskanal (135) zu steuern.

4. Urethroskop (100) nach Anspruch 3, wobei das Verarbeitungsmodul (315) ferner umfasst:
eine Steuervorrichtung, die umfasst:
ein Eingangs- und Ausgangsschnittstellenmodul (405), das konfiguriert ist, um das digitale LDR-Signal von dem LDR (310) zu empfangen;
eine erste Vielzahl von Eingangs- und Ausgangsschnittstellenmodulen (410), die konfiguriert ist, um die digitalen Steuersignale von der Vielzahl von Steuerschaltern (130) zu empfangen;
ein Entscheidungsmodul (415), das konfiguriert ist, um das Vorhandensein der Laserfaser (140) in dem Arbeitskanal (135) auf Grundlage des digitalen LDR-Signals zu bestimmen und eine Art der Zuführbewegung und der Rückzugsbewegung der Laserfaser (140) auf Grundlage der digitalen Steuersignale zu bestimmen; und
eine zweite Vielzahl von Eingangs- und Ausgangsschnittstellenmodulen (420), die mit dem Entscheidungsmodul (415) verbunden ist und konfiguriert ist, um ein Ausgangsdatensignal von dem Entscheidungsmodul (415) zu empfangen und das digitale Motorsignal zu erzeugen.

5. Urethroskop (100) nach Anspruch 3 oder Anspruch 4, wobei die Anfangsposition ein vordefinierter Abstand der Laserfaser (140) in dem Arbeitskanal (135) vor der Zuführbewegung der Laserfaser (140) durch das Laserfaserzufuhrvorrichtungs- und Rückzugsmodul (305) ist.

6. Urethroskop (100) nach Anspruch 1, wobei die Laserfaser (140) konfiguriert ist, um auf ein Leistungsniveau und ein Frequenzniveau erregt zu werden, um das Stäuben des Zielsteins zu ermöglichen.

7. Prozessorlesbares Medium, das Anweisungen umfasst, die, wenn sie von dem Verarbeitungsmodul (315) des Urethroskops (100) nach einem der Ansprüche 3 bis 5 ausgeführt werden, das Verarbeitungsmodul (315) dazu veranlassen, ein Verfahren zum Steuern der Laserfaser in dem Urethroskop (100) durchzuführen, wobei das Verfahren umfasst:
Empfangen eines digitalen lichtabhängigen Widerstandssignals von dem LDR (310) und digitaler Steuersignale von der Vielzahl von Steuerschaltern (130), wobei das digitale LDR-Signal als Reaktion auf das Erfassen des Vorhandenseins der Laserfaser (140) in dem Arbeitskanal (135) der flexiblen Sonde (105) des Urethroskops (100) erzeugt wird;
Verarbeiten des digitalen LDR-Signals und der digitalen Steuersignale, um ein analoges Motorsignal zu erzeugen; und
Initiieren der Aktivierung des Motors (325) in dem Laserfaserzufuhrvorrichtungs- und Rückzugsmodul (305) als Reaktion auf das analoge Motorsignal, um eine von der Zuführbewegung und der Rückzugsbewegung der Laserfaser (140) in dem Arbeitskanal (135) zum Stäuben eines Zielsteins zu steuern.

8. Prozessorlesbares Medium nach Anspruch 7, wobei die Zuführbewegung und die Rückzugsbewegung der Laserfaser (140) gesteuert werden durch:
Erzeugen eines Ausgangsdatensignals, das dem digitalen LDR-Signal und den digitalen Steuersignalen entspricht, wobei das Ausgangsdatensignal das Vorhandensein der Laserfaser (140) in dem Arbeitskanal (135) auf Grundlage des digitalen LDR-Signals und der Art der Zuführbewegung und der Rückzugsbewegung der Laserfaser (140) definiert;
Verarbeiten des Ausgangsdatensignals, um das analoge Motorsignal zu erzeugen; und
Initiieren des Antriebs des Motors (325), um eine Anzahl von Schritten auf Grundlage des analogen Motorsignals zu drehen, um die Laserfaser (140) in eine von der Zuführbewegung und der Rückzugsbewegung der Laserfaser (140) unter Verwendung der Vielzahl von Scheiben (330, 335) zu bewegen.

9. Prozessorlesbares Medium nach Anspruch 7 oder Anspruch 8, wobei das Verfahren ferner umfasst:
Bestimmen der Art der Zuführbewegung und der Rückzugsbewegung der Laserfaser (140) auf Grundlage der digitalen Steuersignale, wobei die Art der Zuführbewegung der Laserfaser (140) eine von einer feinen Zufuhr und einer groben Zufuhr der Laserfaser (140) umfasst, wobei die Art der Rückzugsbewegung der Laserfaser (140) eines von einem feinen Rückzug und einem groben Rückzug der Laserfaser (140) umfasst.

## Revendications

1. Urétéroscope (100) pour la pulvérisation de calculs dans une cavité du corps avec une fibre laser (140), l'urétéroscope (100) comprenant :
une sonde flexible (105) comprenant une extrémité proximale (115) et une extrémité distale (120), la sonde flexible (105) comprenant :
un canal opérateur (135) configuré pour contenir la fibre laser (140) dans l'extrémité distale (120) de la sonde flexible (105), dans une position opérationnelle, la fibre laser (140) étant déviée contre un calcul ciblé pour sa pulvérisation, la position opérationnelle étant une distance prédéfinie de la fibre laser (140) depuis l'extrémité distale (120) de la sonde flexible (105), et définie par des paramètres d'énergie laser et la composition du calcul ciblé ; et
une poignée (110) située à proximité de la sonde flexible (105), la poignée (110) comprenant :
une pluralité de commutateurs de commande (130) configurés pour émettre des signaux de commande numériques, les signaux de commande numériques étant configurés pour permettre à un utilisateur de commander un mouvement assisté par moteur de la fibre laser (140) dans le canal opérateur (135), le mouvement assisté par moteur de la fibre laser (140) comprenant un d'un mouvement de déploiement et d'un mouvement de rétraction ; et
et un module de déploiement et de rétraction de fibre laser (305) configuré pour commander un du mouvement de déploiement et du mouvement de rétraction de la fibre laser (140) dans le canal opérateur (135), le mouvement de déploiement de la fibre laser (140) comprenant un mouvement d'avance de la fibre laser (140) dans une première direction de rotation, et le mouvement de rétraction de la fibre laser (140) comprenant un mouvement de recul de la fibre laser (140) dans une deuxième direction de rotation.

2. Urétéroscope (100) selon la revendication 1, la sonde flexible (105) comprenant en outre :
une pointe de déflexion (125) à l'extrémité distale (120) de la sonde flexible (105), configurée pour être déviée, et dévier à son tour la fibre laser (140), la pointe de déflexion (125) étant positionnée à une distance minimum du calcul ciblé ;
un système de visualisation d'image (215) configuré pour visionner la fibre laser (140) déployée depuis la pointe de déflexion (125) et le calcul ciblé ; et
une source de lumière (220) configurée pour fournir de la lumière pour faciliter la visualisation de la fibre laser (140) et du calcul ciblé par le biais du système de visualisation d'image (215).

3. Urétéroscope (100) selon la revendication 1 ou la revendication 2, le module de déploiement et de rétraction d'une fibre laser (305) comprenant :
une résistance dépendant de la lumière, LDR, (310) configurée pour générer un signal LDR numérique en réponse à la détection de la présence de la fibre laser (140) dans le canal opérateur (135), la fibre laser (140) étant configurée pour être introduite manuellement dans le canal opérateur (135), et positionnée dans une position initiale ;
un module de traitement (315) couplé à la LDR (310), et configuré pour générer un signal moteur numérique en réponse au signal LDR numérique et aux signaux de commande numériques ;
un pilote (320) couplé au module de traitement (315) et configuré pour générer un signal moteur analogique en réponse au signal moteur numérique ;
un moteur (325) couplé au pilote (320) et configuré pour tourner un certain nombre de paliers sur la base du signal moteur analogique ; et
une pluralité de poulies (330, 335) couplées au moteur (320), et configurées pour le contrôle du mouvement assisté par moteur de la fibre laser (140) dans le canal opérateur (135).

4. Urétéroscope (100) selon la revendication 3, le module de traitement (315) comprenant en outre :
une commande comprenant :
un module d'interface d'entrée et de sortie (405) configuré pour recevoir le signal LDR numérique de la LDR (310) ;
une première pluralité de modules d'interface d'entrée et de sortie (410) configurés pour recevoir les signaux de commande numériques de la pluralité de commutateurs de commande (130) ;
un module de prise de décision (415) configuré pour déterminer la présence de la fibre laser (140) dans le canal opérateur (135) basé sur signal LDR numérique, et pour déterminer un type de mouvement de déploiement et de mouvement de rétraction de la fibre laser (140) sur la base des signaux de commande numériques ; et
une deuxième pluralité de modules d'interface d'entrée et de sortie (420) couplés au module de prise de décision (415), et configurée pour recevoir un signal de données de sortie du module de prise de décision (415), et pour générer le signal moteur numérique.

5. Urétéroscope (100) selon la revendication 3 ou la revendication 4, la position initiale étant une distance prédéfinie de la fibre laser (140) dans le canal opérateur (135) préalablement au mouvement de déploiement de la fibre laser (140) par le module de déploiement et de rétraction d'une fibre laser (305).

6. Urétéroscope (100) selon la revendication 1, la fibre laser (140) étant configurée pour être excitée à un niveau de puissance et un niveau de fréquence lui permettant de pulvériser le calcul ciblé.

7. Support lisible par processeur comprenant des instructions qui, lorsqu'elles sont exécutées parle module de traitement (315) de l'urétéroscope (100) selon une quelconque des revendications 3 à 5, engendre l'exécution, par le module de traitement (315), d'une méthode de contrôle de la fibre laser dans l'urétéroscope (100), la méthode comprenant :
la réception d'un signal de résistance dépendant de la lumière numérique de la LDR (310) et de signaux de commande numériques de la pluralité de commutateurs de commande (130), le signal LDR numérique étant généré en réponse à la détection de la présence de la fibre laser (140) dans le canal opérateur (135) de la sonde flexible (105) de l'urétéroscope (100) ;
le traitement du signal LDR numérique et du signal de commande numérique pour générer un signal moteur analogique ; et
le lancement de l'activation du moteur (325) dans le module de déploiement et de rétraction d'une fibre laser (305) en réponse au signal moteur analogique pour commander un du mouvement de déploiement et du mouvement de rétraction de la fibre laser (140) dans le canal opérateur (135) pour la pulvérisation du calcul ciblé.

8. Support lisible par processeur selon la revendication 7, le mouvement de déploiement et le mouvement de rétraction de la fibre laser (140) étant commandés par :
la génération d'un signal de données de sortie correspondant au signal LDR numérique et aux signaux de commande numériques, le signal de données de sortie définissant la présence de la fibre laser (140) dans le canal opérateur (135) sur la base du signal LDR numérique et du type de mouvement de déploiement et de mouvement de rétraction de la fibre laser (140) ;
le traitement du signal de données de sortie pour générer le signal moteur analogique ; et
le lancement de l'entraînement du moteur (325) pour tourner un certain nombre de paliers sur la base du signal moteur analogique pour déplacer la fibre laser (140) dans un du mouvement de déploiement et du mouvement de rétraction de la fibre laser (140) en utilisant la pluralité de poulies (330, 335).

9. Support lisible par processeur selon la revendication 7 ou la revendication 8, la méthode comprenant en outre :
la détermination du type de mouvement de déploiement et de mouvement de rétraction de la fibre laser (140) sur la base des signaux de commande numériques, du type de mouvement de déploiement de la fibre laser (140), comprenant un d'un déploiement de précision et d'un déploiement grossier de la fibre laser (140), du type de mouvement de rétraction de la fibre laser (140) comprenant une d'une rétraction de précision et d'une rétraction grossière de la fibre laser (140).
